# EUROPÄISCHE PATENTSCHRIFT

(11) **EP 0 956 826 B1**
(45) Veröffentlichungstag und Bekanntmachung des Hinweises auf die Patenterteilung: **24.08.2005**
(21) Anmeldenummer: 99107996.3
(22) Anmeldetag: 22.04.1999
(51) Int. Cl.: A61B 18/14

(54) **Hochfrequenzchirurgie-Instrument mit einem Fluidzuführungskanal**
Electrosurgery device with fluid channel
Dispositif électrochirurgical avec conduit pour fluides

(30) Priorität: 11.05.1998 DE 19820995
(43) Veröffentlichungstag der Anmeldung: 17.11.1999
(73) Patentinhaber: Integra ME GmbH, 78532 Tuttlingen (DE)
(72) Erfinder: Hofmann, Helge, 78576 Emmingen (DE); Fritzsch, Gernod, 78532 Tuttlingen (DE)
(74) Vertreter: Manitz, Finsterwald & Partner GbR

(56) Entgegenhaltungen:
- WO-A1-97/16127
- DE-A1- 4 017 626
- DE-A1- 4 440 158
- US-A- 5 609 151
- US-A- 5 643 197

## Beschreibung

Die Erfindung betrifft ein Hochfrequenzchirurgie-Instrument nach dem Oberbegriff des Patentanspruchs 1 (vgl. US-A-5,643,197). Insbesondere befaßt sich die Erfindung mit solchen Hochfrequenzchirurgie-Instrumenten, die zur Koagulation von Biogewebe mit Hochfrequenzenergie bestimmt sind.

Bei der Koagulation von Biogewebe mit Hochfrequenzenergie wird das Gewebe infolge des Stromflusses erwärmt und die Blutstillung wird durch einen komplexen thermisch aktivierten biochemischen Wirkmechanismus erreicht. Dieser Prozeß beruht hauptsächlich auf der thermischen Umwandlung der Eiweiße im Blut ab ca. 60°C sowie der Blutgerinnung. Nachteilig ist dabei, daß Gewebe und Blut an den Koagulationselektroden ankleben, wodurch das soeben erst verschlossene Gefäß im Biogewebe wieder aufreißen kann und durch die Widerstandserhöhung der Stromfluß für die nächste Koagulation behindert wird. Dieses Ankleben wird von mehreren Faktoren beeinflußt; es ist umso stärker, je größer die Elektrodenerwärmung ist.

Aus dem Buch "Hochfrequenz- und Lasertechnik in der Medizin" von Reidenbach, Springer-Verlag, 1983, Seiten 148 bis 167, ist bereits ein Verfahren der Hydrothermosation bekannt, bei dem destilliertes Wasser oder eine physiologische Kochsalzlösung aus Bohrungen von hohlen Koagulationselektroden austritt und durch die dabei eintretende Kühlwirkung bzw. die Trennung von Elektrode und Gewebe der Klebeeffekt reduziert wird. Nachteilig an diesem bekannten Verfahren ist einerseits die Ungleichmäßigkeit der Flüssigkeitsabgabe im Bereich der Behandlungselektrode bzw. die große Menge an Flüssigkeit, die für eine Salzstrahl-HF-Koagulation erforderlich ist und die unweigerlich durch ihre Kühlwirkung die Koagulation selbst erschwert.

Dieses Prinzip wurde auch schon zum Anspülen einer bipolaren Koagulationspinzette genutzt (DE-OS 44 40 158). Nachteilig ist dabei, daß auch hier die Gleichmäßigkeit der Flüssigkeitsabgabe unzureichend ist und die Spülflüssigkeit nicht während der Koagulation zwischen Gewebe und Branchen der Pinzetten austreten kann.

Aus der DE-PS 42 12 053 ist es bereits bekannt, eine Hartstoffschicht auf den Elektroden vorzusehen, die jedoch nur eine unzureichende Wirksamkeit hat, da sich die elektrische Impedanz der Elektroden erhöht und dadurch höhere elektrische Spannungen vom Hochfrequenzgenerator abgegeben werden, die das Ankleben wieder verstärken. Ähnlich nachteilig verhalten sich auch Antihaftschichten auf Teflonbasis oder Siliziumbeschichtungen (US-PS 5 549 604).

Das Ziel der vorliegenden Erfindung besteht darin, ein Hochfrequenzchirurgie-Instrument der eingangs genannten Gattung zu schaffen, bei dem das nachteilige Ankleben von Biogewebe beim Koagulieren weitgehend vermieden wird, ohne daß der Koagulationsvorgang selbst beeinträchtigt wird.

Zur Lösung dieser Aufgabe sind die Merkmale des kennzeichnenden Teils des Patentanspruchs 1 vorgesehen.

Erfindungsgemäß erfolgt also die Flüssigkeitszuführung zur eigentlichen Koagulationsfläche äußerst fein verteilt und gleichmäßig durch einen fluid-, insbesondere flüssigkeitsdurchlässigen porösen Sinterwerkstoff hindurch, der vorzugsweise aus einem biologisch unbedenklichen, elektrisch leitfähigem Material, z.B. rostfreiem Stahl oder Titan besteht.

Die Zuführung der elektrischen Hochfrequenzenergie kann entweder durch eine elektrisch leitende Ausbildung von Schaft, durch Zuleitungen oder durch ein elektrisch leitendes Fluid erfolgen.

Zum Zwecke der Auswechslung einer schadhaften Behandlungselektrode oder auch zu Reinigungs- und Sterilisierungszwecken ist die Ausführungsform nach Anspruch 3 zweckmäßig.

Eine zumindest teilweise aus Sinterwerkstoff bestehende Behandlungselektrode kann auch bei bipolaren Anordnungen Verwendung finden.

Der einfachen Herstellung halber können die erfindungsgemäßen Sinterelektroden sich auch über Bereiche erstrecken, die nicht mit dem Biogewebe in Kontakt kommen. In diesem Falle ist es zweckmäßig, die Poren des Sinterwerkstoffes teilweise zu verschließen.

Für die Zuführung des Fluids ist die Weiterbildung nach Anspruch 8 vorteilhaft.

Die Aufbringung des Sinterwerkstoffs kann nach einem besonders bevorzugten Ausführungsbeispiel gemäß Anspruch 9 erfolgen.

Die Erfindung wird im folgenden beispielhaft anhand der Zeichnung beschrieben; in dieser zeigt:
- Figur 1: eine schematische Ansicht eines nicht beanspruchten monopolaren Hochfrequenzchirurgie-Instrumentes mit geradlinigem Fluidzuführkanal,
- Figur 2: eine entsprechende Ansicht einer nicht beanspruchten Ausführungsform mit gekrümmtem Fluidzuführkanal und einem abgewandelten Koagulationsaufsatz,
- Figur 3: eine schematische Schnittansicht eines nicht beanspruchten und als monopolare Einstichnadelelektrode ausgebildeten Hochfrequenzchirurgie-Instrumentes mit zylinderförmigem Koagulationsaufsatz,
- Figur 4: eine vergrößerte Teilansicht des Bereiches IV in Figur 3,
- Figur 5: den Elektrodenbereich eines nicht beanspruchten Hochfrequenzchirurgie-Instrumentes mit formschlüssiger Befestigung eines die Behandlungselektrode darstellenden Sinterkörpers,
- Figur 6: eine Seitenansicht eines als bipolare Pinzette ausgebildeten Hochfrequenzchirurgie-Instrumentes gemäß der Erfindung,
- Figur 7: eine um 90° um die Längsachse 25 der Pinzette nach Figur 6 gedrehte Ansicht,
- Figur 8: eine vergrößerte Schnittansicht des Elektrodenbereiches der Branchen der Pinzette nach den Figuren 6, 7,
- Figur 9: eine Draufsicht des Gegenstandes von Figur 8 bei abgenommenem Sinterplättchen,
- Figur 10: eine vergrößerte Schnittansicht einer weiteren Ausführungsform des Elektrodenbereichs der Branchen der Pinzetten nach den Figuren 6, 7,
- Figur 11 1: eine Draufsicht des Gegenstandes der Figur 10 bei abgenommenem Sinterplättchen,
- Figur 12: eine Draufsicht auf eine weitere Ausführungsform des Elektrodenbereiches der Branchen der Pinzetten nach den Figuren 6, 7 bei abgenommenem Sinterplättchen,
- Figur 13: eine Schnittansicht nach Linie XIII-XIII in Figur 12, wobei zusätzlich das in Figur 12 weggelassene Sinterplättchen gezeigt ist, und
- Figur 14: eine vergrößerte Schnittansicht des Elektrodenbereiches der Branchen der Pinzette nach den Figuren 6, 7, wobei zusätzlich die lösbare Verbindung des Elektrodenbereiches mit den Branchen der Pinzette nach den Figuren 6, 7 wiedergegeben ist.

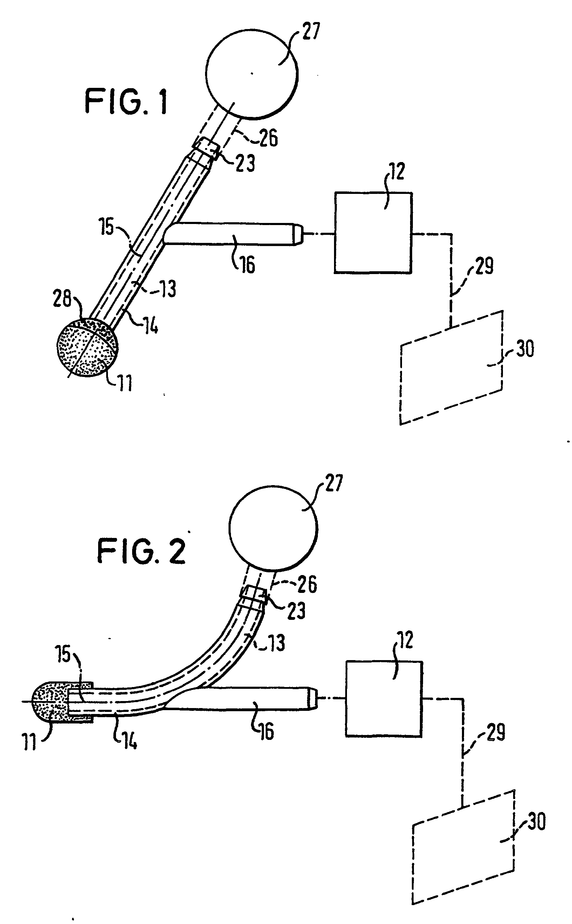

Gemäß Figur 1 ist in einem hohlen Metallschaft 14 mit einer Längsachse 15 ein geradliniger Fluidzuführungskanal 13 vorgesehen, der am patientenfernen Ende mit einem Schlauchanschluß 23 versehen ist, der über einen nur gestrichelt angedeuteten Schlauch 26 mit einer unter Druck stehenden Flüssigkeitsquelle 27 verbunden ist.

Von dem hohlen Metallschaft 14 zweigt unter einem Winkel von 45° ein metallischer Anschluß 16 für den elektrischen Anschluß des hohlen Schaftes 14 an einen Hochfrequenzgenerator 12 ab. Das Anschlußstück 16 ist bevorzugt in einem nicht dargestellten Elektrodenhandgriff befestigt.

Am patientennahen Ende des hohlen Schaftes 14 ist eine kugelförmige Behandlungselektrode 11 befestigt, die aus einem fluiddurchlässigen Sinterwerkstoff, insbesondere Sintermetall besteht. Die Poren des Sinterkörpers 11 sind nach vorne und zur Seite offen, jedoch nach hinten durch eine Verschlußschicht 28 so verschlossen, daß die durch den Fluidzufuhrkanal 13 zugeführte Flüssigkeit nur aus dem vorderen und seitlichen Bereich der Behandlungselektrode 11 austreten kann.

Der hohle Schaft 14 ragt bis in den Sinterkörper 11 hinein und ist mit diesem verschweißt. Die Verschlußschicht 28 kann durch Aufbringen einer geeigneten Substanz oder aber auch durch mechanische Bearbeitung der Rückseite des Sinterkörpers 11 gebildet werden.

Insbesondere wenn der kugelförmige Behandlungselektroden-Sinterkörper 11 aus nicht leitendem Keramikmaterial besteht, soll als Spülflüssigkeit eine physiologische, 0,9 %ige Kochsalzlösung verwendet werden.

Die Behandlungselektrode 11 nach Figur 1 ist eine monopolare Elektrode. Aus diesem Grunde ist an dem Hochfrequenzgenerator 12 über eine gestrichelt dargestellte Leitung 29 eine am Körper des Patienten anzubringende Neutralelektrode 30 angeschlossen.

In den folgenden Figuren bezeichnen gleiche Bezugszahlen entsprechende Bauteile wie im Ausführungsbeispiel nach Figur 1.

Beim Ausführungsbeispiel nach Figur 2 ist der hohle Schaft 14 mit dem Fluidzuführungskanal 13 abgebogen, während das Anschlußstück 16 in einer geraden Linie mit dem hier als zylindrischer Koagulationsaufsatz 11 mit abgerundetem vorderen Ende ausgebildeten Behandlungselektrode liegt.

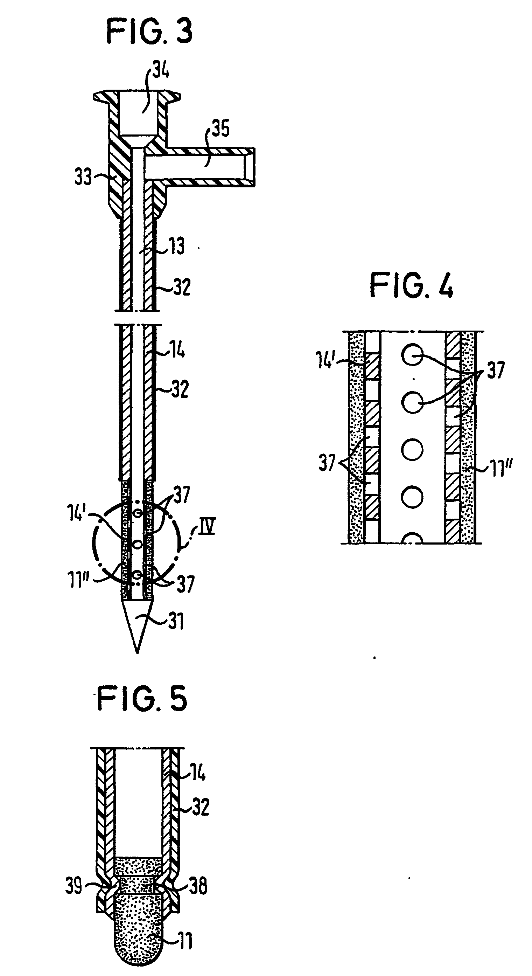

Figur 3 zeigt eine monopolare Einstichnadelelektrode, wie sie z.B. zur Tumordenaturierung verwendet wird. Hinter der metallischen Spitze 31 ist auf einem Bereich 14' verringerten Durchmessers des hohlen Schaftes ein dünner rohrförmiger Formkörper 11'' aus Sinterwerkstoff angeordnet, während der dahinterliegende Teil des hohlen Schaftes 14 mit einer Isolierbeschichtung 32 versehen ist. Am patientenfernen Ende ist ein Kunststoff-Formteil 33 vorgesehen, in dem ein Luer-Lock-Anschluß 34 untergebracht ist. An dem Kunststoff-Formteil 33 mündet seitlich eine Hochfrequenz-Anschlußbuchse 35, in die ein nicht gezeigter Hochfrequenz-Anschlußstecker eingesteckt werden kann, der eine elektrische Verbindung zwischen dem nicht dargestellten Hochfrequenzgenerator und dem hohlen Metallschaft 14 herzustellen gestattet. Zentral im Inneren der Einstichnadelelektrode nach Figur 3 befindet sich wieder der Fluidzuführungskanal 13.

Der Bereich 14' verringerten Außendurchmesser weist eine Anzahl von Radialbohrungen 37 auf, wie man besonders gut in Fig. 4 erkennen kann. Durch diese kann die bei 34 zugeführte Flüssigkeit von innen in den Sinter-Formkörper 11'' gelangen, der zweckmäßig durch Plasmaflammspritzen als Beschichtung auf den Bereich 14' aufgebracht ist. Durch die Aufbringung des porösen Materials als Beschichtung auf den verjüngten Endteil des mit Bohrungen 37 versehenen hohlen Metallschaftes 14 wird das an sich brüchige Sintermaterial großflächig so gut abgestützt, daß beim Gebrauch eine Beschädigung der Sinterbeschichtung wirksam vermieden wird. Die Beschichtung 11'' besteht bevorzugt aus porösem rostfreien Stahl.

Beim Gebrauch der erfindungsgemäßen Behandlungselektroden 11 wird außer der Spannungszufuhr durch den Hochfrequenzgenerator 12 auch Flüssigkeit, vorzugsweise Wasser durch den Flüssigkeitskanal 13 zugeführt, welche großflächig aus den Poren des Sintermaterials 11, 11'' austritt und somit ein Ankleben des behandelten Biogewebes an der Behandlungselektrode verhindert.

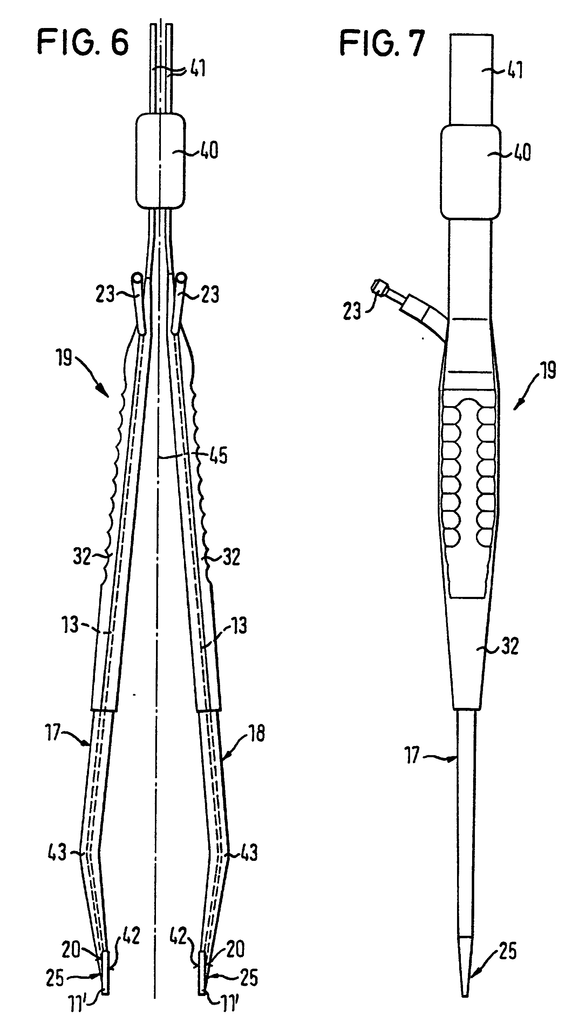

Nach Figur 5 besteht die als Sinterkörper ausgebildete Behandlungselektrode 11 ebenso wie beim Ausführungsbeispiel nach Figur 2 aus einem zylindrischen, vorne abgerundeten Formkörper, der jedoch rückseitig mit einer Umfangsnut 38 versehen ist, wodurch das in den vorne offenen hohlen Metallschaft 14 eingeführte, hintere Ende der Behandlungselekrode 11 durch eine im Bereich der Umfangsnut rundum angebrachte Sicke 39 fest am patientennahen Ende des Metallschaftes 14 befestigt werden kann.

Nach den Figuren 6 und 7 sind die beiden Branchen 17, 18 einer Koagulationspinzette 19 an ihrem patientenfernen Ende durch einen Isolierkörper 40 elektrisch voneinander isoliert mechanisch miteinander verbunden. Hinten ragen aus dem Isolierkörper 40 die für die Zuführung von Hochfrequenzenergie vorgesehenen Kontakte 41 heraus. In oder an den Branchen 17, 18 befindet sich je ein Fluidzuführungskanal 13, welcher im hinteren Bereich in jeweils einem Schlauchanschluß 23 mündet.

Im hinteren und mittleren Bereich sind die Branchen 17, 18 mit einer Isolierbeschichtung 32 versehen.

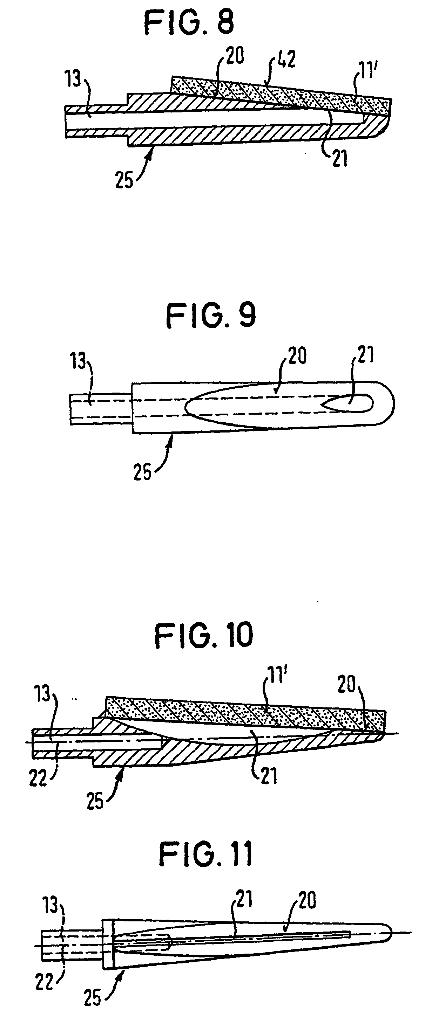

Wie man besonders gut den Figuren 8 und 9 entnehmen kann, sind die beiden Branchen 17, 18 im patientennahen Elektrodenbereich 25 angeschrägt, so daß eine leicht nach außen geneigte ebene Endfläche 20 entsteht, wobei der Fluidzuführungskanal 13 in einer Fluidaustrittsöffnung 21 in der angeschrägten Endfläche 20 mündet.

Die angeschrägte Endfläche 20 ist nach den Figuren 6, 7 und 8 mit einem planparallelen Plättchen 11' aus Sinterwerkstoff bedeckt, welches vorzugsweise aufgeschweißt ist. Auf diese Weise wird die durch den Fluidzufuhrkanal 13 zugeführte Flüssigkeit von der angeschrägten Fläche 20 her durch die Fluidaustrittsöffnung 21 in das Sinterplättchen 11' gedrückt, wo sie sich aufgrund der zahlreichen feinen Poren gleichmäßig verteilt und gleichmäßig von der äußeren eigentlichen Behandlungsfläche 42 austritt.

Nach Figur 6 sind die Branchen 17, 18 nahe dem Elektrodenbereich 25 bei 43 etwas nach innen derart abgeknickt, daß die auf die schrägen Endflächen 20 aufgesetzten planparallelen Sinterplättchen 11' zumindest im wesentlichen zueinander parallele Behandlungsflächen 42 aufweisen.

Die Figuren 10 und 11 zeigen gegenüber den Figuren 8 und 9 eine etwas andere Ausbildung der Fluidaustrittsöffnung 21, welche hier als Schlitz 21 ausgebildet ist, der sich über annähernd die gesamte Länge des Sinterplättchens 11' erstreckt, so daß das Sinterplättchen 11' von innen her über eine größere Länge mit Flüssigkeit beaufschlagt wird als bei der Ausführungsform nach den Figuren 8, 9. Der Schlitz 21 verläuft zumindest im wesentlichen parallel zur Achse 22 des Elektrodenbereiches 25.

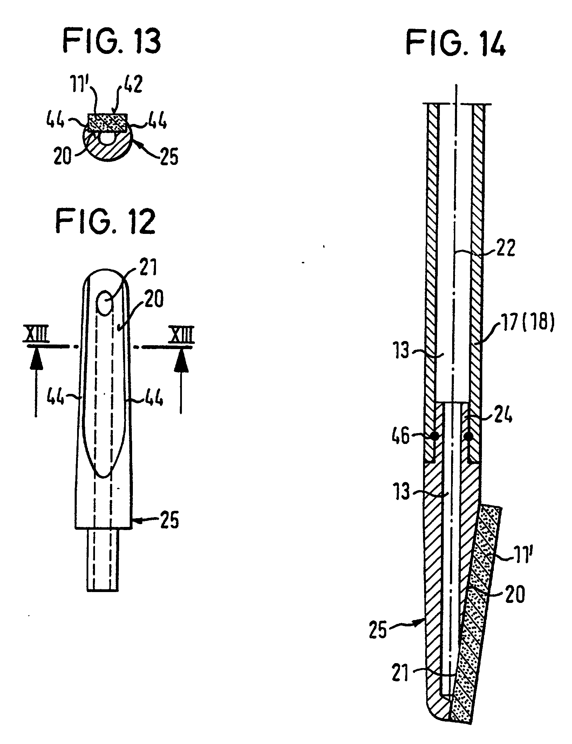

Bei der Ausführungsform nach den Figure 12 und 13 ist die schräge Endfläche 20 des Elektrodenbereiches 25 als Grund einer Nut ausgebildet, deren seitliche Stege 44 das aufgebrachte Sinterplättchen 11' auch seitlich abstützen, so daß es gegen Beschädigungen beim Gebrauch besonders gut geschützt ist.

Das Sinterplättchen 11' nach Figur 13 kann in die Nut eingepreßt, an den Rändern verschweißt oder anderweitig, z.B. durch Verkleben befestigt sein.

Der Elektrodenbereich 25 nach Figur 14, der entsprechend auch bei den Ausführungen nach den Fig. 8 bis 12 vorhanden ist, ist lösbar mit den Branchen 17, 18 der Koagulationspinzette 19 verbunden. Hierzu weist der Elektrodenbereich 25, der das Ende des Fluidzuführungskanals 13 enthält, an seinem rückwärtigen Ende einen koaxialen Anschlußstutzen 24 verringerten Durchmessers auf, der einen Außendurchmesser entsprechend dem Durchmesser des in den Branchen 17, 18 enthaltenen Fluidzuführungskanals 13 besitzt. Am Umfang des Anschlußstutzens 14 und in der radial gegenüberliegenden Wand der hohlen Branchen 17, 18 befinden sich Umfangsnuten, in welche als Dicht- und Schnappelement ein O-Ring 46 eingelegt ist. Auf diese Weise kann der Elektrodenbereich 25 gegen eine Rastkraft von den Branchen 17, 18 axial abgezogen und in entgegengesetzter Richtung eingerastet werden. Sowohl zu Reinigungs- und Sterilisierungszwecken als auch zur Reparatur oder zum Auswechseln beschädigter Sinterplättchen 11' kann somit der Elektrodenbereich 25 von den Branchen 17, 18 der Koagulationspinzette 19 abgenommen werden. Die Porengröße des Sinterwerkstoffes liegt zwischen 0,5 und 150 µm.

### Bezugszeichenliste

- 11: Behandlungselektrode
- 11': Plättchen
- 11'': Beschichtung
- 12: Hochfrequenzgenerator
- 13: Fluidzufuhrkanal
- 14: Schaft
- 14': verjüngter Bereich des Schaftes
- 15: Längsachse
- 16: Anschluß für Hochfrequenzenergiezuführung
- 17: Branche
- 18: Branche
- 19: Koagulationspinzette
- 20: angeschrägte Endfläche
- 21: Fluidaustrittsöffnung
- 22: Elektrodenbereichs-Achse
- 23: Schlauchanschluß
- 24: Anschlußstutzen
- 25: Elektrodenbereich
- 26: Schlauch
- 27: Flüssigkeitsquelle
- 28: Verschlußstück
- 29: Leitung
- 30: Neutralelektrode
- 31: Spitze
- 32: Isolierbeschichtung
- 33: Kunststoff-Formteil
- 34: Luer-Lock-Anschluß
- 35: Hochfrequenz-Anschlußbuchse
- 36: Stromzuführung
- 37: Radialbohrungen
- 38: Umfangsnut
- 39: Sicke
- 40: Isolierkörper
- 41: Kontakte
- 42: Behandlungsfläche
- 43: Abknickung
- 44: O-Ring
- 45: Längsmittelachse der Koagulationspinzette

## Patentansprüche

1. Hochfrequenzchirurgie-Instrument mit wenigstens einer an einen Hochfrequenzgenerator (12) anschließbaren bzw. angeschlossenen Behandlungselektrode (11, 11', 11''), die zumindest teilweise fluiddurchlässig ist, und der durch wenigstens einen in einem hohlen Schaft (14) vorgesehenen Fluidzuführungskanal (13) ein dem Ankleben des Biogewebes an der Behandlungselektrode (11) entgegenwirkendes Fluid, insbesondere eine Flüssigkeit, zuführbar bzw. zugeführt ist, wobei mindestens der Bereich (11') der Behandlungselektrode, der zur Wechselwirkung mit dem Biogewebe bestimmt ist, ganz oder teilweise aus einem fluiddurchlässigen, porösen, biologisch unbedenklichen Sinterwerkstoff besteht,
**dadurch gekennzeichnet,**
**daß** der Sinterwerkstoff als Plättchen (11') geformt ist und mit einer flachen Seite auf dem mit wenigstens einer Fluidaustrittsöffnung (21) versehenen angeschrägten Ende (20) des hohlen Schaftes (14) dicht befestigt ist.

2. Hochfrequenzchirurgie-Instrument nach Anspruch 1,
**dadurch gekennzeichnet,**
**daß** der hohle Schaft (14) elektrisch leitend ist und insbesondere aus Metall besteht.

3. Hochfrequenzchirurgie-Instrument nach Anspruch 1,
**dadurch gekennzeichnet,**
**daß** die Behandlungselektrode (11) lösbar am Schaft befestigt ist.

4. Hochfrequenzchirurgie-Instrument nach einem der vorhergehenden Ansprüche,
**dadurch gekennzeichnet,**
**daß** zwei Behandlungselektroden (11) als bipolare Anordnung vorgesehen sind, welche bevorzugt an den Spitzen der einen hohlen Schaft bildenden Branchen (17, 18) einer bipolaren Koagulationspinzette (19) ausgebildet sind.

5. Hochfrequenzchirurgie-Instrument nach einem der vorhergehenden Ansprüche,
**dadurch gekennzeichnet, daß** das Plättchen (11') planparallel ist und bevorzugt im wesentlichen die Form der Schnittfläche des angeschrägten Endes (20) hat.

6. Hochfrequenzchirurgie-Instrument nach einem der vorhergehenden Ansprüche,
**dadurch gekennzeichnet,**
**daß** die Fluidaustrittsöffnung (21) als zumindest im wesentlichen parallel zur Achse (15) des hohlen Schaftes (14) bzw. zur Branchenachse (22) verlaufender Schlitz (21) ausgebildet ist.

7. Hochfrequenzchirurgie-Instrument nach einem der vorhergehenden Ansprüche,
**dadurch gekennzeichnet,**
**daß** die Poren des Sinterwerkstoffes der Behandlungselektrode (11, 11', 11'') zumindest im Bereich der Oberfläche an bestimmten Stellen, wo keine Berührung mit dem Biogewebe zu erwarten ist und/oder kein Fluid austreten soll, z.B. durch mechanische Bearbeitung oder durch Klebstoff bzw. Lot verschlossen sind.

8. Hochfrequenzchirurgie-Instrument nach einem der vorstehenden Ansprüche,
**dadurch gekennzeichnet,**
**daß** der hohle Schaft (14) bzw. die Branchen (17, 18) an der von der Behandlungselektrode (11, 11') abgewandten Seite einen Schlauchanschluß (23) besitzen.

9. Hochfrequenzchirurgie-Instrument nach einem der vorhergehenden Ansprüche,
**dadurch gekennzeichnet,**
**daß** der Sinterwerkstoff als Beschichtung (11'') z.B. galvanisch oder durch Plasmaspritzen auf einen hohlen, mit Austrittsöffnungen (37) versehenen Trägerkörper (14') aufgebracht ist.

10. Hochfrequenzchirurgie-Instrument nach einem der vorhergehenden Ansprüche,
**dadurch gekennzeichnet,**
**daß** der Sinterwerkstoff ein Sintermetall ist und leitend mit einem Hochfrequenzanschluß (16) verbunden ist.

11. Hochfrequenzchirurgie-Instrument nach einem der Ansprüche 1 bis 9,
**dadurch gekennzeichnet,**
**daß** der Sinterwerkstoff Keramik ist.

12. Hochfrequenzchirurgie-Instrument nach einem der vorhergehenden Ansprüche,
**dadurch gekennzeichnet,**
**daß** das Fluid eine elektrisch leitende Flüssigkeit, z.B. eine physiologische Kochsalzlösung ist.

## Claims

1. High frequency surgical instrument comprising at least one treatment electrode (11, 11', 11") which can be or is connected to a high frequency generator (12), which is at least partly fluid-permeable and to which a fluid, in particular a liquid, which counteracts the sticking of the biological tissue to the treatment electrode (11) can be or is fed through at least one liquid infeed passage (13) provided in a hollow shaft (14), wherein at least the region (11') of the treatment electrode which is intended for the interaction with the biological tissue consists totally or partly of a liquid-permeable, porous, biologically unobjectionable sinter material,
**characterized in that**
the sinter material is formed as a platelet (11') and is sealingly secured at a flat side to the chamfered end (20) of the hollow shaft (14) provided with at least one fluid discharge opening (21).

2. High frequency surgical instrument in accordance with claim 1, **characterized in that** the hollow shaft (14) is electrically conductive and in particular consists of metal.

3. High frequency surgical instrument in accordance with claim 1, **characterized in that** the treatment electrode (11) is releasably fastened to the shaft.

4. High frequency surgical instrument in accordance with any one of the preceding claims, **characterized in that** two treatment electrodes (11), which are preferably formed at the tips of the branches (17, 18) of bipolar coagulation forceps (19) forming a hollow shaft, are provided as a bipolar arrangement.

5. High frequency surgical instrument in accordance with any one of the preceding claims, **characterized in that** the platelet (11') is planar parallel and preferably has substantially the shape of the cutting area of the chamfered end (20).

6. High frequency surgical instrument in accordance with any one of the preceding claims, **characterized in that** the fluid discharge opening (21) is made as a slit (21) which extends at least substantially parallel to the axis (15) of the hollow shaft (14) or to the branch axis (22) respectively.

7. High frequency surgical instrument in accordance with any one of the preceding claims, **characterized in that** the pores of the sinter material of the treatment electrode (11, 11', 11") are closed, e.g. by mechanical processing or by an adhesive or solder, at least in the region of the surface at specific locations where no contact with the biological tissue is to be expected and/or no fluid is to be discharged.

8. High frequency surgical instrument in accordance with any one of the preceding claims, **characterized in that** the hollow shaft (14) or the branches (17, 18) respectively have a hose connection (23) at the end remote from the treatment electrode (11, 11').

9. High frequency surgical instrument in accordance with any one of the preceding claims, **characterized in that** the sinter material is applied as a coating (11"), e.g. galvanically or by plasma spraying, to a hollow carrier body (14') which is provided with discharge openings (37).

10. High frequency surgical instrument in accordance with any one of the preceding claims, **characterized in that** the sinter material is a sinter metal and is conductively connected to a high frequency connector (16).

11. High frequency surgical instrument in accordance with any one of the claims 1 to 9, **characterized in that** the sinter material is a ceramic material.

12. High frequency surgical instrument in accordance with any one of the preceding claims, **characterized in that** the fluid is an electrically conductive liquid, e.g. a physiological saline solution.

## Revendications

1. Instrument chirurgical à haute fréquence, comprenant au moins une électrode de traitement (11, 11', 11") raccordée ou susceptible d'être raccordée à un générateur haute fréquence (12), ladite électrode étant au moins partiellement perméable aux fluides et un fluide, en particulier un liquide, s'opposant à l'adhérence du tissu biologique sur l'électrode de traitement (11), est amené ou susceptible d'être amené à ladite électrode via au moins un canal d'amenée de fluide (13) prévu dans une tige creuse (14), dans lequel au moins la région (11') de l'électrode de traitement qui est destinée à coopérer avec le tissu biologique est constituée en totalité ou en partie en un matériau fritté perméable aux fluides, poreux et biologiquement compatible,
**caractérisé en ce que** le matériau fritté est formé sous forme de plaquette (11') et est fixé de façon étanche par une face plane sur l'extrémité (20), de la tige creuse (14), biseautée et pourvue d'au moins une ouverture de sortie de fluide (21).

2. Instrument chirurgical à haute fréquence selon la revendication 1, **caractérisé en ce que** la tige creuse (14) est conductrice de l'électricité, et est en particulier réalisée en métal.

3. Instrument chirurgical à haute fréquence selon la revendication 1, **caractérisé en ce que** l'électrode de traitement (11) est fixée sur la tige de façon détachable.

4. Instrument chirurgical à haute fréquence selon l'une des revendications précédentes, **caractérisé en ce qu'**il est prévu deux électrodes de traitement (11) sous forme d'un agencement bipolaire, lesdites électrodes étant de préférence réalisées au niveau des pointes des branches (17, 18), formant une tige creuse, d'une pincette de coagulation bipolaire (19).

5. Instrument chirurgical à haute fréquence selon l'une des revendications précédentes, **caractérisé en ce que** la plaquette (11') est à faces planes parallèles et a de préférence sensiblement la forme de la surface de coupe de l'extrémité biseautée (20).

6. Instrument chirurgical à haute fréquence selon l'une des revendications précédentes, **caractérisé en ce que** l'ouverture de sortie de fluide (21) est réalisée comme une fente (21) qui s'étend au moins sensiblement parallèlement à l'axe (15) de la tige de creuse (14) ou parallèlement à l'axe (22) des branches.

7. Instrument chirurgical à haute fréquence selon l'une des revendications précédentes, **caractérisé en ce que** les pores du matériau fritté de l'électrode de traitement (11, 11', 11") sont obturés au moins dans la région de la surface à certains emplacements auxquels on ne s'attend à aucun contact avec le tissu biologique et/ou aucun fluide ne doit sortir, par exemple par usinage mécanique, ou au moyen d'une colle ou d'une brasure.

8. Instrument chirurgical à haute fréquence selon l'une des revendications précédentes, **caractérisé en ce que** la tige creuse (14) ou les branches (17, 18) possède(nt) un raccord pour tuyau (23) sur le côté détourné de l'électrode de traitement (11, 11').

9. Instrument chirurgical à haute fréquence selon l'une des revendications précédentes, **caractérisé en ce que** le matériau fritté est appliqué sous forme d'un revêtement (11"), par exemple par voie galvanique ou par pulvérisation au plasma sur un corps porteur (14') creux pourvu d'ouvertures de sortie (37).

10. Instrument chirurgical à haute fréquence selon l'une des revendications précédentes, **caractérisé en ce que** le matériau fritté est un métal fritté est **en ce qu'**il est relié de façon conductrice à une borne à haute fréquence (16).

11. Instrument chirurgical à haute fréquence selon l'une des revendications 11 9, **caractérisé en ce que** le matériau fritté est de la céramique.

12. Instrument chirurgical à haute fréquence selon l'une des revendications précédentes, **caractérisé en ce que** le fluide est un liquide conducteur de l'électricité, par exemple une solution de chlorure de sodium physiologique.
